# EUROPÄISCHE PATENTSCHRIFT

(11) **EP 1 115 849 B1**
(45) Veröffentlichungstag und Bekanntmachung des Hinweises auf die Patenterteilung: **01.02.2006**
(21) Anmeldenummer: 99948831.5
(22) Anmeldetag: 22.09.1999
(51) Int. Cl.: C12N 9/90, C12N 9/10, C12N 9/12, C12Q 1/48

(54) **GENE DES 1-DESOXY-D-XYLULOSE-BIOSYNTHESEWEGS**
GENES OF THE 1-DESOXY-D-XYLULOSE BIOSYNTHETIC PATHWAY
VOIE DE SYNTHESE BIOLOGIQUE DES GENES DES 1-DESOXY-D-XYLULOSE

(30) Priorität: 22.09.1998 DE 19843279; 21.05.1999 DE 19923567
(43) Veröffentlichungstag der Anmeldung: 18.07.2001
(73) Patentinhaber: BioAgency AG, 22525 Hamburg (DE)
(72) Erfinder: JOMAA, Hassan Jomaa Pharmaka GmbH, 35392 Giessen (DE)
(74) Vertreter: Dreiss, Fuhlendorf, Steimle & Becker
(86) Internationale Anmeldenummer: PCT/EP1999/007055
(87) Internationale Veröffentlichungsnummer: WO 2000/017233

(56) Entgegenhaltungen:
- WO-A-99/52938
- DE-A- 19 752 700
- PUTRA ET AL: "Incorporation of [2,3-[13]C2]- and [2,4-[13]C2]-D-1-Deoxyxylulose into ubiquinone of Escherichia coli via the Mevalonate-Independent pathway for Isoprenoid Biosynthesis" TETRAHEDRON LETTERS,NL,ELSEVIER SCIENCE PUBLISHERS, AMSTERDAM, Bd. 39, Nr. 39, 1998, Seiten 23-26-26, XP002116676 ISSN: 0040-4039
- KUZUYAMA ET AL: "Direct formation of 2-C-Methyl-D-Erythritol 4-phosphate from 1-Deoxy-D-Xylulose 5-phosphate Reductoisomerase, a new enzyme in the non-mevalonate pathway to isopentenyl diphosphate" TETRAHEDRON LETTERS,NL,ELSEVIER SCIENCE PUBLISHERS, AMSTERDAM, Bd. 39, Nr. 39, 1998, Seiten 4509-4512-44512, XP002116675 ISSN: 0040-4039
- SCHWENDER, J. ET AL.: "Cloning and heterologous expression of a cDNA encoding 1-deoxy-d-xylulose-5-phosphate reductoisomerase of Arabidopsis thaliana" FEBS LETTERS, Bd. 455, Juli 1999 (1999-07), Seiten 140-144, XP002132424
- LANGE ET AL: "A family of transketolases that directs isoprenoid biosynthesis via a mevalonate-independent pathway" FASEB JOURNAL,US,FED. OF AMERICAN SOC. FOR EXPERIMENTAL BIOLOGY, BETHESDA, MD, Bd. 95, März 1998 (1998-03), Seiten 2100-2104, XP002116672 ISSN: 0892-6638
- EMINV DATABASE: "AC: EF111813" PLASMODIUM FALCIPARUM 1-DEOXY-D-XYLULOSE 5-PHOSPHATE REDUCTOISOMERASE, 11. Januar 1999 (1999-01-11), XP002132425
- TREMBL DATABASE: "AC: O96693" PLASMODIUM FALCIPARUM 1-DEOXY-D-XYLULOSE 5-PHOSPHATE REDUCTOISOMERASE, 1. Mai 1999 (1999-05-01), XP002132426
- SPRENGER ET AL: "Identification of a thiamin-dependent synthase in Escherichia coli required for the formation of the 1-deoxy-D-xylulose 5-phosphate precursor to isoprenoids, thiamin, and pyridoxol" FASEB JOURNAL,US,FED. OF AMERICAN SOC. FOR EXPERIMENTAL BIOLOGY, BETHESDA, MD, Bd. 94, November 1997 (1997-11), Seiten 12857-12862, XP002116674 ISSN: 0892-6638
- TREMBL DATABASE: "AC: QZ8H0" CHLAMYDIA PNEUMONIAE GCPE PROTEIN, 1. Mai 1999 (1999-05-01), XP002132427

### SEQUENZPROTOKOLL

<110> Jomaa, Hassan
<120> Gene des 1-Desoxy-D-xylulose-Biosynthesewegs
<130> 15696
<140> PCT/EP99
   <141> 1999-09-22
<150> DE19923567.8
   <151> 1999-05-22
<150> DE19843279.8
   <151> 1998-09-22
<160> 6
<170> Patent In Ver. 2.1
<210> 1
   <211> 1467
   <212> DNA
   <213> Plasmodium falciparum
<220>
   <221> CDS
   <222> (1) .. (1467)
<220>
   <221> gene
   <222> (1)..(1467)
<220>
   <221> mRNA
   <222> (1) .. (1467)
<400> 1
   atg aag aaa tat att tat ata tat ttt ttc ttc atc aca ata act att    48
   Met Lys Lys Tyr Ile Tyr Ile Tyr Phe Phe Phe Ile Thr Ile Thr Ile
    1               5                  10                  15
   aat gat tta gta ata aat aat aca tca aaa tgt gtt tcc att gaa aga    96
   Asn Asp Leu Val Ile Asn Asn Thr Ser Lys Cys Val Ser Ile Glu Arg
                20                  25                  30
   aga aaa aat aac gca tat ata aat tat ggt ata gga tat aat gga cca   144
   Arg Lys Asn Asn Ala Tyr Ile Asn Tyr Gly Ile Gly Tyr Asn Gly Pro
            35                  40                  45
   gat aat aaa ata aca aag agt aga aga tgt aaa aga ata aag tta tgc   192
   Asp Asn Lys Ile Thr Lys Ser Arg Arg Cys Lys Arg Ile Lys Leu Cys
        50                  55                  60
   aaa aag gat tta ata gat att ggt gca ata aag aaa cca att aat gta   240
   Lys Lys Asp Leu Ile Asp Ile Gly Ala Ile Lys Lys Pro Ile Asn Val
    65                  70                  75                  80
   gca att ttt gga agt act ggt agt ata ggt acg aat gct tta aat ata   288
   Ala Ile Phe Gly Ser Thr Gly Ser Ile Gly Thr Asn Ala Leu Asn Ile
                    85                  90                  95
   ata agg gag tgt aat aaa att gaa aat gtt ttt aat gtt aaa gca ttg   336
   Ile Arg Glu Cys Asn Lys Ile Glu Asn Val Phe Asn Val Lys Ala Leu
               100                 105                 110
   tat gtg aat aag agt gtg aat gaa tta tat gaa caa gct aga gaa ttt   384
   Tyr Val Asn Lys Ser Val Asn Glu Leu Tyr Glu Gln Ala Arg Glu Phe
           115                 120                 125
   tta cca gaa tat ttg tgt ata cat gat aaa agt gta tat gaa gaa tta   432
   Leu Pro Glu Tyr Leu Cys Ile His Asp Lys Ser Val Tyr Glu Glu Leu
       130                 135                 140
   aaa gaa ctg gta aaa aat ata aaa gat tat aaa cct ata ata ttg tgt   480
   Lys Glu Leu Val Lys Asn Ile Lys Asp Tyr Lys Pro Ile Ile Leu Cys
   145                 150                 155                 160
   ggt gat gaa ggg atg aaa gaa ata tgt agt agt aat agt ata gat aaa   528
   Gly Asp Glu Gly Met Lys Glu Ile Cys Ser Ser Asn Ser Ile Asp Lys
                   165                 170                 175
   ata gtt att ggt att gat tct ttt caa gga tta tat tct act atg tat   576
   Ile Val Ile Gly Ile Asp Ser Phe Gln Gly Leu Tyr Ser Thr Met Tyr
               180                 185                 190
   gca att atg aat aat aaa ata gtt gcg tta gct aat aaa gaa tcc att     624
   Ala Ile Met Asn Asn Lys Ile Val Ala Leu Ala Asn Lys Glu Ser Ile
           195                 200                 205
   gtc tct gct ggt ttc ttt tta aag aaa tta tta aat att cat aaa aat     672
   Val Ser Ala Gly Phe Phe Leu Lys Lys Leu Leu Asn Ile His Lys Asn
       210                 215                 220
   gca aag ata ata cct gtt gat tca gaa cat agt gct ata ttt caa tgt     720
   Ala Lys Ile Ile Pro Val Asp Ser Glu His Ser Ala Ile Phe Gln Cys
   225                 230                 235                 240
   tta gat aat aat aag gta tta aaa aca aaa tgt tta caa gac aat ttt     768
   Leu Asp Asn Asn Lys Val Leu Lys Thr Lys Cys Leu Gln Asp Asn Phe
                   245                 250                 255
   tct aaa att aac aat ata aat aaa ata ttt tta tgt tca tct gga ggt     816
   Ser Lys Ile Asn Asn Ile Asn Lys Ile Phe Leu Cys Ser Ser Gly Gly
               260                 265                 270
   cca ttt caa aat tta act atg gac gaa tta aaa aat gta aca tca gaa     864
   Pro Phe Gln Asn Leu Thr Met Asp Glu Leu Lys Asn Val Thr Ser Glu
           275                 280                 285
   aat gct tta aag cat cct aaa tgg aaa atg ggt aag aaa ata act ata     912
   Asn Ala Leu Lys His Pro Lys Trp Lys Met Gly Lys Lys Ile Thr Ile
       290                 295                 300
   gat tct gca act atg atg aat aaa ggt tta gag gtt ata gaa acc cat     960
   Asp Ser Ala Thr Met Met Asn Lys Gly Leu Glu Val Ile Glu Thr His
   305                 310                 315                 320
   ttt tta ttt gat gta gat tat aat gat ata gaa gtt ata gta cat aaa    1008
   Phe Leu Phe Asp Val Asp Tyr Asn Asp Ile Glu Val Ile Val His Lys
                   325                 330                 335
   gaa tgc att ata cat tct tgt gtt gaa ttt ata gac aaa tca gta ata    1056
   Glu Cys Ile Ile His Ser Cys Val Glu Phe Ile Asp Lys Ser Val Ile
               340                 345                 350
   agt caa atg tat tat cca gat atg caa ata ccc ata tta tat tct tta    1104
   Ser Gln Met Tyr Tyr Pro Asp Met Gln Ile Pro Ile Leu Tyr Ser Leu
           355                 360                 365
   aca tgg cct gat aga ata aaa aca aat tta aaa cct tta gat ttg gct    1152
   Thr Trp Pro Asp Arg Ile Lys Thr Asn Leu Lys Pro Leu Asp Leu Ala
       370                 375                 380
   cag gtt tca act ctt aca ttt cat aaa cct tct tta gaa cat ttc ccg    1200
   Gln Val Ser Thr Leu Thr Phe His Lys Pro Ser Leu Glu His Phe Pro
   385                 390                 395                 400
   tgt att aaa tta gct tat caa gca ggt ata aaa gga aac ttt tat cca    1248
   Cys Ile Lys Leu Ala Tyr Gln Ala Gly Ile Lys Gly Asn Phe Tyr Pro
                   405                 410                 415
   act gta cta aat gcg tca aat gaa ata gct aac aac tta ttt ttg aat    1296
   Thr Val Leu Asn Ala Ser Asn Glu Ile Ala Asn Asn Leu Phe Leu Asn
               420                 425                 430
   aat aaa att aaa tat ttt gat att tcc tct ata ata tcg caa gtt ctt    1344
   Asn Lys Ile Lys Tyr Phe Asp Ile Ser Ser Ile Ile Ser Gln Val Leu
           435                 440                 445
   gaa tct ttc aat tct caa aag gtt tcg gaa aat agt gaa gat tta atg    1392
   Glu Ser Phe Asn Ser Gln Lys Val Ser Glu Asn Ser Glu Asp Leu Met
       450                 455                 460
   aag caa att cta caa ata cat tct tgg gcc aaa gat aaa gct acc gat    1440
   Lys Gln Ile Leu Gln Ile His Ser Trp Ala Lys Asp Lys Ala Thr Asp
   465                 470                 475                 480
   ata tac aac aaa cat aat tct tca tag                                1467
   Ile Tyr Asn Lys His Asn Ser Ser
                   485
<210> 2
   <211> 488
   <212> PRT
   <213> Plasmodium falciparum
<400> 2
   Met Lys Lys Tyr Ile Tyr Ile Tyr Phe Phe Phe Ile Thr Ile Thr Ile
   1 5 10 15
   Asn Asp Leu Val Ile Asn Asn Thr Ser Lys Cys Val Ser Ile Glu Arg
   20 25 30
   Arg Lys Asn Asn Ala Tyr Ile Asn Tyr Gly Ile Gly Tyr Asn Gly Pro
   35 40 45
   Asp Asn Lys Ile Thr Lys Ser Arg Arg Cys Lys Arg Ile Lys Leu Cys
   50 55 60
   Lys Lys Asp Leu Ile Asp Ile Gly Ala Ile Lys Lys Pro Ile Asn Val
   65 70 75 80
   Ala Ile Phe Gly Ser Thr Gly Ser Ile Gly Thr Asn Ala Leu Asn Ile
   85 90 95
   Ile Arg Glu Cys Asn Lys Ile Glu Asn Val Phe Asn Val Lys Ala Leu
   100 105 110
   Tyr Val Asn Lys Ser Val Asn Glu Leu Tyr Glu Gln Ala Arg Glu Phe
   115 120 125
   Leu Pro Glu Tyr Leu Cys Ile His Asp Lys Ser Val Tyr Glu Glu Leu
   130 135 140
   Lys Glu Leu Val Lys Asn Ile Lys Asp Tyr Lys Pro Ile Ile Leu Cys
   145 150 155 160
   Gly Asp Glu Gly Met Lys Glu Ile Cys Ser Ser Asn Ser Ile Asp Lys
   165 170 175
   Ile Val Ile Gly Ile Asp Ser Phe Gln Gly Leu Tyr Ser Thr Met Tyr
   180 185 190
   Ala Ile Met Asn Asn Lys Ile Val Ala Leu Ala Asn Lys Glu Ser Ile
   195 200 205
   Val Ser Ala Gly Phe Phe Leu Lys Lys Leu Leu Asn Ile His Lys Asn
   210 215 220
   Ala Lys Ile Ile Pro Val Asp Ser Glu His Ser Ala Ile Phe Gln Cys
   225 230 235 240
   Leu Asp Asn Asn Lys Val Leu Lys Thr Lys Cys Leu Gln Asp Asn Phe
   245 250 255
   Ser Lys Ile Asn Asn Ile Asn Lys Ile Phe Leu Cys Ser Ser Gly Gly
   260 265 270
   Pro Phe Gln Asn Leu Thr Met Asp Glu Leu Lys Asn Val Thr Ser Glu
   275 280 285
   Asn Ala Leu Lys His Pro Lys Trp Lys Met Gly Lys Lys Ile Thr Ile
   290 295 300
   Asp Ser Ala Thr Met Met Asn Lys Gly Leu Glu Val Ile Glu Thr His
   305 310 315 320
   Phe Leu Phe Asp Val Asp Tyr Asn Asp Ile Glu Val Ile Val His Lys
   325 330 335
   Glu Cys Ile Ile His Ser Cys Val Glu Phe Ile Asp Lys Ser Val Ile
   340 345 350
   Ser Gln Met Tyr Tyr Pro Asp Met Gln Ile Pro Ile Leu Tyr Ser Leu
   355 360 365
   Thr Trp Pro Asp Arg Ile Lys Thr Asn Leu Lys Pro Leu Asp Leu Ala
   370 375 380
   Gln Val Ser Thr Leu Thr Phe His Lys Pro Ser Leu Glu His Phe Pro
   385 390 395 400
   Cys Ile Lys Leu Ala Tyr Gln Ala Gly Ile Lys Gly Asn Phe Tyr Pro
   405 410 415
   Thr Val Leu Asn Ala Ser Asn Glu Ile Ala Asn Asn Leu Phe Leu Asn
   420 425 430
   Asn Lys Ile Lys Tyr Phe Asp Ile Ser Ser Ile Ile Ser Gln Val Leu
   435 440 445
   Glu Ser Phe Asn Ser Gln Lys Val Ser Glu Asn Ser Glu Asp Leu Met
   450 455 460
   Lys Gln Ile Leu Gln Ile His Ser Trp Ala Lys Asp Lys Ala Thr Asp
   465 470 475 480
   Ile Tyr Asn Lys His Asn Ser Ser
   485
<210> 3
   <211> 3872
   <212> DNA
   <213> Plasmodium falciparum
<220>
   <221> CDS
   <222> (126)..(3740)
<220>
   <221> gene
   <222> (1)..(3870)
<220>
   <221> mRNA
   <222> (1)..(3870)
<400> 3
   ggtaatatac gtataatata tatataatat attcttacgt atgtatcatt tatgaatcat 60
   aataatattc taaatttacc ttccgttttt gctcgatctt ctcattttcg tttcagcttt 120
   tatca atg att ttt aat tat gtg ttt ttt aag aac ttt gta cca gtt gtt 170
   Met Ile Phe Asn Tyr Val Phe Phe Lys Asn Phe Val Pro Val Val
   1 5 10 15
   cta tac att ctc ctt ata ata tat att aac tta aat ggc atg aat aat 218
   Leu Tyr Ile Leu Leu Ile Ile Tyr Ile Asn Leu Asn Gly Met Asn Asn
   20 25 30
   aaa aat caa ata aaa aca gaa aaa att tat ata aag aaa ttg aat agg   266
   Lys Asn Gln Ile Lys Thr Glu Lys Ile Tyr Ile Lys Lys Leu Asn Arg
               35                  40                  45
   ttg tca agg aaa aat tcg tta tgt agt tct aaa aat aaa ata gca tgc   314
   Leu Ser Arg Lys Asn Ser Leu Cys Ser Ser Lys Asn Lys Ile Ala Cys
           50                  55                  60
   ttg ttc gat ata gga aat gat gat aat aga aat acg aca tat ggc tat   362
   Leu Phe Asp Ile Gly Asn Asp Asp Asn Arg Asn Thr Thr Tyr Gly Tyr
       65                  70                  75
   aat gtg aat gtt aaa aat gat gat att aat tcc tta cta aaa aat aat   410
   Asn Val Asn Val Lys Asn Asp Asp Ile Asn Ser Leu Leu Lys Asn Asn
   80                  85                  90                  95
   tat agt aat aaa ttg tac atg gat aag agg aaa aat att aat aat gta   458
   Tyr Ser Asn Lys Leu Tyr Met Asp Lys Arg Lys Asn Ile Asn Asn Val
                   100                 105                 110
   att agt act aat aaa ata tct ggg tcc att tca aat att tgt agt aga   506
   Ile Ser Thr Asn Lys Ile Ser Gly Ser Ile Ser Asn Ile Cys Ser Arg
               115                 120                 125
   aat caa aaa gaa aat gaa caa aaa aga aat aaa caa aga tgt tta act   554
   Asn Gln Lys Glu Asn Glu Gln Lys Arg Asn Lys Gln Arg Cys Leu Thr
           130                 135                 140
   caa tgt cac act tat aat atg tca cat gaa cag gac aaa cta gct aat   602
   Gln Cys His Thr Tyr Asn Met Ser His Glu Gln Asp Lys Leu Ala Asn
       145                 150                 155
   gat aat aat agg aat aat aaa aag aat ttt aat tta tta ttt ata aat   650
   Asp Asn Asn Arg Asn Asn Lys Lys Asn Phe Asn Leu Leu Phe Ile Asn
   160                 165                 170                 175
   tat ttt aat ttg aaa cga atg aaa aat tct ctt cta aat aaa gac aat   698
   Tyr Phe Asn Leu Lys Arg Met Lys Asn Ser Leu Leu Asn Lys Asp Asn
                   180                 185                 190
   ttc ttt tac tgt aaa gaa aaa aaa ttg tca ttt ctg cat aag gcc tat   746
   Phe Phe Tyr Cys Lys Glu Lys Lys Leu Ser Phe Leu His Lys Ala Tyr
               195                 200                 205
   aaa aaa aaa aat tgc act ttt caa aat tat agt tta aaa aga aaa tct    794
   Lys Lys Lys Asn Cys Thr Phe Gln Asn Tyr Ser Leu Lys Arg Lys Ser
           210                 215                 220
   aat cgt gat tca cat aaa ttg ttt tct gga gaa ttt gac gat tat aca    842
   Asn Arg Asp Ser His Lys Leu Phe Ser Gly Glu Phe Asp Asp Tyr Thr
       225                 230                 235
   aat aat aat gct tta tat gaa tcc gaa aaa aaa gaa tac att aca cta    890
   Asn Asn Asn Ala Leu Tyr Glu Ser Glu Lys Lys Glu Tyr Ile Thr Leu
   240                 245                 250                 255
   aat aat aat aat aaa aat aat aat aat aaa aat aat gat aat aaa aat    938
   Asn Asn Asn Asn Lys Asn Asn Asn Asn Lys Asn Asn Asp Asn Lys Asn
                   260                 265                 270
   aat gat aat aat gat tat aat aat aat aat agt tgt aat aat tta gga    986
   Asn Asp Asn Asn Asp Tyr Asn Asn Asn Asn Ser Cys Asn Asn Leu Gly
               275                 280                 285
   gag aga tcc aat cat tat gat aat tat ggt gga gat aat aat aat cca   1034
   Glu Arg Ser Asn His Tyr Asp Asn Tyr Gly Gly Asp Asn Asn Asn Pro
           290                 295                 300
   tgt aat aat aat aat gac aaa tat gat ata gga aaa tat ttc aaa cag   1082
   Cys Asn Asn Asn Asn Asp Lys Tyr Asp Ile Gly Lys Tyr Phe Lys Gln
       305                 310                 315
   att aat acc ttt att aat att gat gaa tat aaa act ata tat ggt gat   1130
   Ile Asn Thr Phe Ile Asn Ile Asp Glu Tyr Lys Thr Ile Tyr Gly Asp
   320                 325                 330                 335
   gaa ata tat aaa gaa ata tat gaa cta tat gta gaa aga aat att cct   1178
   Glu Ile Tyr Lys Glu Ile Tyr Glu Leu Tyr Val Glu Arg Asn Ile Pro
                   340                 345                 350
   gaa tat tat gaa cga aaa tat ttt tca gaa gat att aaa aag agt gtc   1226
   Glu Tyr Tyr Glu Arg Lys Tyr Phe Ser Glu Asp Ile Lys Lys Ser Val
               355                 360                 365
   cta ttt gat ata gat aaa tat aat gat gtc gaa ttt gaa aaa gct ata   1274
   Leu Phe Asp Ile Asp Lys Tyr Asn Asp Val Glu Phe Glu Lys Ala Ile
           370                 375                 380
   aaa gaa gaa ttt ata aat aat gga gtt tat att aat aat ata gat aat   1322
   Lys Glu Glu Phe Ile Asn Asn Gly Val Tyr Ile Asn Asn Ile Asp Asn
       385                 390                 395
   aca tat tat aaa aaa gaa aat att tta ata atg aaa aag ata tta cat   1370
   Thr Tyr Tyr Lys Lys Glu Asn Ile Leu Ile Met Lys Lys Ile Leu His
   400                 405                 410                 415
   tat ttc cca tta tta aaa tta att aat aat cca tca gat tta aaa aag   1418
   Tyr Phe Pro Leu Leu Lys Leu Ile Asn Asn Pro Ser Asp Leu Lys Lys
                   420                 425                 430
   tta aaa aaa caa tat tta cct tta tta gca cat gaa tta aaa ata ttt   1466
   Leu Lys Lys Gln Tyr Leu Pro Leu Leu Ala His Glu Leu Lys Ile Phe
               435                 440                 445
   tta ttt ttt att gta aat ata aca gga ggt cat ttt tcc tct gtt tta   1514
   Leu Phe Phe Ile Val Asn Ile Thr Gly Gly His Phe Ser Ser Val Leu
           450                 455                 460
   agc tct tta gaa att caa tta tta tta ttg tat att ttt aat caa cca   1562
   Ser Ser Leu Glu Ile Gln Leu Leu Leu Leu Tyr Ile Phe Asn Gln Pro
       465                 470                 475
   tat gat aat gtt ata tat gat ata gga cat caa gca tat gta cat aag   1610
   Tyr Asp Asn Val Ile Tyr Asp Ile Gly His Gln Ala Tyr Val His Lys
   480                 485                 490                 495
   ata ttg acc gga aga aaa cta tta ttt cta tca tta aga aat aaa aaa   1658
   Ile Leu Thr Gly Arg Lys Leu Leu Phe Leu Ser Leu Arg Asn Lys Lys
                   500                 505                 510
   ggt att agt gga ttc cta aat att ttt gaa agt att tat gat aaa ttt   1706
   Gly Ile Ser Gly Phe Leu Asn Ile Phe Glu Ser Ile Tyr Asp Lys Phe
               515                 520                 525
   ggg gct ggt cac agt tcc act tca tta agt gct ata caa gga tat tat   1754
   Gly Ala Gly His Ser Ser Thr Ser Leu Ser Ala Ile Gln Gly Tyr Tyr
   530 535 540
   gaa gcc gag tgg caa gtg aag aat aaa gaa aaa tat gga aat gga gat 1802
   Glu Ala Glu Trp Gln Val Lys Asn Lys Glu Lys Tyr Gly Asn Gly Asp
   545 550 555
   ata gaa ata agt gat aac gca aat gtc acg aat aat gaa agg ata ttt 1850
   Ile Glu Ile Ser Asp Asn Ala Asn Val Thr Asn Asn Glu Arg Ile Phe
   560 565 570 575
   caa aaa gga ata cac aat gat aat aat att aac aat aat att aat aat 1898
   Gln Lys Gly Ile His Asn Asp Asn Asn Ile Asn Asn Asn Ile Asn Asn
   580 585 590
   aat aat tat atc aat cct tca gat gtg gta gga aga gaa aat acg aat 1946
   Asn Asn Tyr Ile Asn Pro Ser Asp Val Val Gly Arg Glu Asn Thr Asn
   595 600 605
   gta cca aat gta cga aat gat aac cat aac gtg gat aaa gta cac att 1994
   Val Pro Asn Val Arg Asn Asp Asn His Asn Val Asp Lys Val His Ile
   610 615 620
   gct att ata gga gat ggt ggt tta aca ggt gga atg gca tta gaa gcg 2042
   Ala Ile Ile Gly Asp Gly Gly Leu Thr Gly Gly Met Ala Leu Glu Ala
   625 630 635
   tta aat tat att tca ttc ttg aat tct aaa att tta att att tat aat 2090
   Leu Asn Tyr Ile Ser Phe Leu Asn Ser Lys Ile Leu Ile Ile Tyr Asn
   640 645 650 655
   gat aac gga caa gtt tct tta cca aca aat gcc gta agt ata tca ggt 2138
   Asp Asn Gly Gln Val Ser Leu Pro Thr Asn Ala Val Ser Ile Ser Gly
   660 665 670
   aat aga cct ata ggt tct ata tca gat cat tta cat tat ttt gtt tct 2186
   Asn Arg Pro Ile Gly Ser Ile Ser Asp His Leu His Tyr Phe Val Ser
   675 680 685
   aat ata gaa gca aat gct ggt gat aat aaa tta tcg aaa aat gca aaa 2234
   Asn Ile Glu Ala Asn Ala Gly Asp Asn Lys Leu Ser Lys Asn Ala Lys
   690 695 700
   gag aat aac att ttt gaa aat ttg aat tat gat tat att ggt gtt gtg 2282
   Glu Asn Asn Ile Phe Glu Asn Leu Asn Tyr Asp Tyr Ile Gly Val Val
   705 710 715
   aat ggt aat aat aca gaa gag ctc ttt aaa gta tta aat aat ata aaa 2330
   Asn Gly Asn Asn Thr Glu Glu Leu Phe Lys Val Leu Asn Asn Ile Lys
   720 725 730 735
   gaa aat aaa tta aaa aga gct act gtt ctt cat gta cgt aca aaa aaa 2378
   Glu Asn Lys Leu Lys Arg Ala Thr Val Leu His Val Arg Thr Lys Lys
   740 745 750
   tcg aat gat ttt ata aat tca aag agt cca ata agt ata ttg cac tct 2426
   Ser Asn Asp Phe Ile Asn Ser Lys Ser Pro Ile Ser Ile Leu His Ser
   755 760 765
   ata aag aaa aat gag att ttc cct ttc gat acc act ata tta aat gga 2474
   Ile Lys Lys Asn Glu Ile Phe Pro Phe Asp Thr Thr Ile Leu Asn Gly
   770 775 780
   aat att cat aag gag aac aag ata gaa gaa gag aaa aat gtg tct tca 2522
   Asn Ile His Lys Glu Asn Lys Ile Glu Glu Glu Lys Asn Val Ser Ser
   785 790 795
   tct aca aag tat gat gta aat aat aag aat aat aaa aat aat gat aat 2570
   Ser Thr Lys Tyr Asp Val Asn Asn Lys Asn Asn Lys Asn Asn Asp Asn
   800 805 810 815
   agt gaa att ata aaa tat gaa gat atg ttt tca aaa gag acg ttc aca 2618
   Ser Glu Ile Ile Lys Tyr Glu Asp Met Phe Ser Lys Glu Thr Phe Thr
   820 825 830
   gat ata tat aca aat gaa atg tta aaa tat tta aag aaa gat aga aat 2666
   Asp Ile Tyr Thr Asn Glu Met Leu Lys Tyr Leu Lys Lys Asp Arg Asn
   835 840 845
   ata ata ttc cta tct ccc gct atg tta gga gga tca gga ttg gtt aaa 2714
   Ile Ile Phe Leu Ser Pro Ala Met Leu Gly Gly Ser Gly Leu Val Lys
   850 855 860
   att agt gag cgt tat cca aat aat gta tat gat gta ggt ata gca gaa 2762
   Ile Ser Glu Arg Tyr Pro Asn Asn Val Tyr Asp Val Gly Ile Ala Glu
       865                 870                 875
   caa cat tct gta act ttc gca gca gct atg gca atg aat aag aaa tta     2810
   Gln His Ser Val Thr Phe Ala Ala Ala Met Ala Met Asn Lys Lys Leu
   880                 885                 890                 895
   aaa ata caa tta tgt ata tat tcg acc ttt tta caa aga gca tat gat     2858
   Lys Ile Gln Leu Cys Ile Tyr Ser Thr Phe Leu Gln Arg Ala Tyr Asp
                   900                 905                 910
   caa att ata cat gat ctt aat tta caa aat ata cct tta aag gtt ata     2906
   Gln Ile Ile His Asp Leu Asn Leu Gln Asn Ile Pro Leu Lys Val Ile
               915                 920                 925
   att gga aga agt gga tta gta gga gag gat ggg gca aca cat caa ggt     2954
   Ile Gly Arg Ser Gly Leu Val Gly Glu Asp Gly Ala Thr His Gln Gly
           930                 935                 940
   ata tat gat tta tct tat ctt ggg aca ctt aac aat gca tat ata ata     3002
   Ile Tyr Asp Leu Ser Tyr Leu Gly Thr Leu Asn Asn Ala Tyr Ile Ile
       945                 950                 955
   tct cca agt aat caa gtt gat ttg aaa aga gct ctt agg ttt gct tat     3050
   Ser Pro Ser Asn Gln Val Asp Leu Lys Arg Ala Leu Arg Phe Ala Tyr
   960                 965                 970                 975
   tta gat aag gac cat tct gtg tat ata cgt ata ccc aga atg aac ata     3098
   Leu Asp Lys Asp His Ser Val Tyr Ile Arg Ile Pro Arg Met Asn Ile
                   980                 985                 990
   tta agt gat aag tac atg aaa gga tat ttg aac att cat atg aaa aat     3146
   Leu Ser Asp Lys Tyr Met Lys Gly Tyr Leu Asn Ile His Met Lys Asn
               995                 1000                1005
   gag agc aaa aat atc gat gta aac gtg gat ata aac gat gat gta gat     3194
   Glu Ser Lys Asn Ile Asp Val Asn Val Asp Ile Asn Asp Asp Val Asp
           1010                1015                1020
   aaa tat agt gaa gaa tat atg gac gat gat aat ttt ata aaa tcg ttt     3242
   Lys Tyr Ser Glu Glu Tyr Met Asp Asp Asp Asn Phe Ile Lys Ser Phe
       1025                1030                1035
   att gga aaa tct aga att att aaa atg gat aat gaa aat aat aat aca    3290
   Ile Gly Lys Ser Arg Ile Ile Lys Met Asp Asn Glu Asn Asn Asn Thr
   1040                1045                1050                1055
   aat gaa cat tat tca agc aga gga gat aca cag aca aaa aaa aaa aaa    3338
   Asn Glu His Tyr Ser Ser Arg Gly Asp Thr Gln Thr Lys Lys Lys Lys
                   1060                1065                1070
   gtt tgt atc ttt aac atg ggt agt atg ctt ttt aat gta att aat gct    3386
   Val Cys Ile Phe Asn Met Gly Ser Met Leu Phe Asn Val Ile Asn Ala
               1075                1080                1085
   ata aaa gaa att gaa aaa gaa caa tat att tca cat aat tat tct ttt    3434
   Ile Lys Glu Ile Glu Lys Glu Gln Tyr Ile Ser His Asn Tyr Ser Phe
           1090                1095                1100
   tca att gtt gat atg ata ttt tta aat cct tta gat aaa aat atg ata    3482
   Ser Ile Val Asp Met Ile Phe Leu Asn Pro Leu Asp Lys Asn Met Ile
       1105                1110                1115
   gat cat gta ata aaa caa aat aaa cat caa tat tta att act tat gaa    3530
   Asp His Val Ile Lys Gln Asn Lys His Gln Tyr Leu Ile Thr Tyr Glu
   1120                1125                1130                1135
   gat aat act ata ggt ggt ttt tct aca cat ttc aat aat tat tta ata    3578
   Asp Asn Thr Ile Gly Gly Phe Ser Thr His Phe Asn Asn Tyr Leu Ile
                   1140                1145                1150
   gaa aat aat tat att aca aaa cat aac tta tat gtt cat aat att tat    3626
   Glu Asn Asn Tyr Ile Thr Lys His Asn Leu Tyr Val His Asn Ile Tyr
               1155                1160                1165
   tta tct aat gag cca att gaa cat gca tct ttt aag gat caa caa gaa    3674
   Leu Ser Asn Glu Pro Ile Glu His Ala Ser Phe Lys Asp Gln Gln Glu
           1170                1175                1180
   gtc gtc aaa atg gat aaa tgt agt ctt gtc aat aga att aaa aat tat    3722
   Val Val Lys Met Asp Lys Cys Ser Leu Val Asn Arg Ile Lys Asn Tyr
       1185                1190                1195
   ctt aaa aat aat cct aca tgatgtaaga taaatatata tttctaaaat           3770
   Leu Lys Asn Asn Pro Thr
   1200 1205
   tatttttttt ttatacttta atgtgtacaa taaaatatat atctaaatat attttatttg 3830
   tacgcttttt tttttttttt tttaattgtt atttttgtat at 3872
<210> 4
   <211> 1205
   <212> PRT
   <213> Plasmodium falciparum
<400> 4
   Met Ile Phe Asn Tyr Val Phe Phe Lys Asn Phe Val Pro Val Val Leu
   1 5 10 15
   Tyr Ile Leu Leu Ile Ile Tyr Ile Asn Leu Asn Gly Met Asn Asn Lys
   20 25 30
   Asn Gln Ile Lys Thr Glu Lys Ile Tyr Ile Lys Lys Leu Asn Arg Leu
   35 40 45
   Ser Arg Lys Asn Ser Leu Cys Ser Ser Lys Asn Lys Ile Ala Cys Leu
   50 55 60
   Phe Asp Ile Gly Asn Asp Asp Asn Arg Asn Thr Thr Tyr Gly Tyr Asn
   65 70 75 80
   Val Asn Val Lys Asn Asp Asp Ile Asn Ser Leu Leu Lys Asn Asn Tyr
   85 90 95
   Ser Asn Lys Leu Tyr Met Asp Lys Arg Lys Asn Ile Asn Asn Val Ile
   100 105 110
   Ser Thr Asn Lys Ile Ser Gly Ser Ile Ser Asn Ile Cys Ser Arg Asn
   115 120 125
   Gln Lys Glu Asn Glu Gln Lys Arg Asn Lys Gln Arg Cys Leu Thr Gln
   130 135 140
   Cys His Thr Tyr Asn Met Ser His Glu Gln Asp Lys Leu Ala Asn Asp
   145 150 155 160
   Asn Asn Arg Asn Asn Lys Lys Asn Phe Asn Leu Leu Phe Ile Asn Tyr
   165 170 175
   Phe Asn Leu Lys Arg Met Lys Asn Ser Leu Leu Asn Lys Asp Asn Phe
   180 185 190
   Phe Tyr Cys Lys Glu Lys Lys Leu Ser Phe Leu His Lys Ala Tyr Lys
   195 200 205
   Lys Lys Asn Cys Thr Phe Gln Asn Tyr Ser Leu Lys Arg Lys Ser Asn
   210 215 220
   Arg Asp Ser His Lys Leu Phe Ser Gly Glu Phe Asp Asp Tyr Thr Asn
   225 230 235 240
   Asn Asn Ala Leu Tyr Glu Ser Glu Lys Lys Glu Tyr Ile Thr Leu Asn
   245 250 255
   Asn Asn Asn Lys Asn Asn Asn Asn Lys Asn Asn Asp Asn Lys Asn Asn
   260 265 270
   Asp Asn Asn Asp Tyr Asn Asn Asn Asn Ser Cys Asn Asn Leu Gly Glu
   275 280 285
   Arg Ser Asn His Tyr Asp Asn Tyr Gly Gly Asp Asn Asn Asn Pro Cys
   290 295 300
   Asn Asn Asn Asn Asp Lys Tyr Asp Ile Gly Lys Tyr Phe Lys Gln Ile
   305 310 315 320
   Asn Thr Phe Ile Asn Ile Asp Glu Tyr Lys Thr Ile Tyr Gly Asp Glu
   325 330 335
   Ile Tyr Lys Glu Ile Tyr Glu Leu Tyr Val Glu Arg Asn Ile Pro Glu
   340 345 350
   Tyr Tyr Glu Arg Lys Tyr Phe Ser Glu Asp Ile Lys Lys Ser Val Leu
   355 360 365
   Phe Asp Ile Asp Lys Tyr Asn Asp Val Glu Phe Glu Lys Ala Ile Lys
   370 375 380
   Glu Glu Phe Ile Asn Asn Gly Val Tyr Ile Asn Asn Ile Asp Asn Thr
   385 390 395 400
   Tyr Tyr Lys Lys Glu Asn Ile Leu Ile Met Lys Lys Ile Leu His Tyr
   405 410 415
   Phe Pro Leu Leu Lys Leu Ile Asn Asn Pro Ser Asp Leu Lys Lys Leu
   420 425 430
   Lys Lys Gln Tyr Leu Pro Leu Leu Ala His Glu Leu Lys Ile Phe Leu
   435 440 445
   Phe Phe Ile Val Asn Ile Thr Gly Gly His Phe Ser Ser Val Leu Ser
   450 455 460
   Ser Leu Glu Ile Gln Leu Leu Leu Leu Tyr Ile Phe Asn Gln Pro Tyr
   465 470 475 480
   Asp Asn Val Ile Tyr Asp Ile Gly His Gln Ala Tyr Val His Lys Ile
   485 490 495
   Leu Thr Gly Arg Lys Leu Leu Phe Leu Ser Leu Arg Asn Lys Lys Gly
   500 505 510
   Ile Ser Gly Phe Leu Asn Ile Phe Glu Ser Ile Tyr Asp Lys Phe Gly
   515 520 525
   Ala Gly His Ser Ser Thr Ser Leu Ser Ala Ile Gln Gly Tyr Tyr Glu
   530 535 540
   Ala Glu Trp Gln Val Lys Asn Lys Glu Lys Tyr Gly Asn Gly Asp Ile
   545 550 555 560
   Glu Ile Ser Asp Asn Ala Asn Val Thr Asn Asn Glu Arg Ile Phe Gln
   565 570 575
   Lys Gly Ile His Asn Asp Asn Asn Ile Asn Asn Asn Ile Asn Asn Asn
   580 585 590
   Asn Tyr Ile Asn Pro Ser Asp Val Val Gly Arg Glu Asn Thr Asn Val
   595 600 605
   Pro Asn Val Arg Asn Asp Asn His Asn Val Asp Lys Val His Ile Ala
   610 615 620
   Ile Ile Gly Asp Gly Gly Leu Thr Gly Gly Met Ala Leu Glu Ala Leu
   625 630 635 640
   Asn Tyr Ile Ser Phe Leu Asn Ser Lys Ile Leu Ile Ile Tyr Asn Asp
   645 650 655
   Asn Gly Gln Val Ser Leu Pro Thr Asn Ala Val Ser Ile Ser Gly Asn
   660 665 670
   Arg Pro Ile Gly Ser Ile Ser Asp His Leu His Tyr Phe Val Ser Asn
   675 680 685
   Ile Glu Ala Asn Ala Gly Asp Asn Lys Leu Ser Lys Asn Ala Lys Glu
   690 695 700
   Asn Asn Ile Phe Glu Asn Leu Asn Tyr Asp Tyr Ile Gly Val Val Asn
   705 710 715 720
   Gly Asn Asn Thr Glu Glu Leu Phe Lys Val Leu Asn Asn Ile Lys Glu
   725 730 735
   Asn Lys Leu Lys Arg Ala Thr Val Leu His Val Arg Thr Lys Lys Ser
   740 745 750
   Asn Asp Phe Ile Asn Ser Lys Ser Pro Ile Ser Ile Leu His Ser Ile
   755 760 765
   Lys Lys Asn Glu Ile Phe Pro Phe Asp Thr Thr Ile Leu Asn Gly Asn
   770 775 780
   Ile His Lys Glu Asn Lys Ile Glu Glu Glu Lys Asn Val Ser Ser Ser
   785 790 795 800
   Thr Lys Tyr Asp Val Asn Asn Lys Asn Asn Lys Asn Asn Asp Asn Ser
   805 810 815
   Glu Ile Ile Lys Tyr Glu Asp Met Phe Ser Lys Glu Thr Phe Thr Asp
   820 825 830
   Ile Tyr Thr Asn Glu Met Leu Lys Tyr Leu Lys Lys Asp Arg Asn Ile
   835 840 845
   Ile Phe Leu Ser Pro Ala Met Leu Gly Gly Ser Gly Leu Val Lys Ile
   850 855 860
   Ser Glu Arg Tyr Pro Asn Asn Val Tyr Asp Val Gly Ile Ala Glu Gln
   865 870 875 880
   His Ser Val Thr Phe Ala Ala Ala Met Ala Met Asn Lys Lys Leu Lys
   885 890 895
   Ile Gln Leu Cys Ile Tyr Ser Thr Phe Leu Gln Arg Ala Tyr Asp Gln
   900 905 910
   Ile Ile His Asp Leu Asn Leu Gln Asn Ile Pro Leu Lys Val Ile Ile
   915 920 925
   Gly Arg Ser Gly Leu Val Gly Glu Asp Gly Ala Thr His Gln Gly Ile
   930 935 940
   Tyr Asp Leu Ser Tyr Leu Gly Thr Leu Asn Asn Ala Tyr Ile Ile Ser
   945 950 955 960
   Pro Ser Asn Gln Val Asp Leu Lys Arg Ala Leu Arg Phe Ala Tyr Leu
   965 970 975
   Asp Lys Asp His Ser Val Tyr Ile Arg Ile Pro Arg Met Asn Ile Leu
   980 985 990
   Ser Asp Lys Tyr Met Lys Gly Tyr Leu Asn Ile His Met Lys Asn Glu
   995 1000 1005
   Ser Lys Asn Ile Asp Val Asn Val Asp Ile Asn Asp Asp Val Asp Lys
   1010 1015 1020
   Tyr Ser Glu Glu Tyr Met Asp Asp Asp Asn Phe Ile Lys Ser Phe Ile
   025 1030 1035 1040
   Gly Lys Ser Arg Ile Ile Lys Met Asp Asn Glu Asn Asn Asn Thr Asn
   1045 1050 1055
   Glu His Tyr Ser Ser Arg Gly Asp Thr Gln Thr Lys Lys Lys Lys Val
   1060 1065 1070
   Cys Ile Phe Asn Met Gly Ser Met Leu Phe Asn Val Ile Asn Ala Ile
   1075 1080 1085
   Lys Glu Ile Glu Lys Glu Gln Tyr Ile Ser His Asn Tyr Ser Phe Ser
   1090 1095 1100
   Ile Val Asp Met Ile Phe Leu Asn Pro Leu Asp Lys Asn Met Ile Asp
   105 1110 1115 1120
   His Val Ile Lys Gln Asn Lys His Gln Tyr Leu Ile Thr Tyr Glu Asp
   1125 1130 1135
   Asn Thr Ile Gly Gly Phe Ser Thr His Phe Asn Asn Tyr Leu Ile Glu
   1140 1145 1150
   Asn Asn Tyr Ile Thr Lys His Asn Leu Tyr Val His Asn Ile Tyr Leu
   1155 1160 1165
   Ser Asn Glu Pro Ile Glu His Ala Ser Phe Lys Asp Gln Gln Glu Val
   1170 1175 1180
   Val Lys Met Asp Lys Cys Ser Leu Val Asn Arg Ile Lys Asn Tyr Leu
   185 1190 1195 1200
   Lys Asn Asn Pro Thr
   1205
<210> 5
   <211> 3147
   <212> DNA
   <213> Plasmodium falciparum
<220>
   <221> CDS
   <222> (199)..(2670)
<400> 5
   tttcattttt ctttacccac atatatatat atatatatat aatatatata tataatatta 60
   tatatttgat atatgattta aaattgtaac ataaaaaaaa taattatatt aaatatgtgt 120
   atacatctcc aacatataaa tattattttt tattattatt tttttttttt tttttcataa 180
   tgcctgaata accacaaa atg agt tat ata aaa aga ctg att ctt ttt atg    231
                       Met Ser Tyr Ile Lys Arg Leu Ile Leu Phe Met
                        1               5                  10
   tta ctg ttt tat tct cat gta aaa att aaa aaa tta ttt att aaa att    279
   Leu Leu Phe Tyr Ser His Val Lys Ile Lys Lys Leu Phe Ile Lys Ile
               15                  20                  25
   tct aat gta aac ata ttt ttt gca gaa gca aag aaa aat gga aaa aag    327
   Ser Asn Val Asn Ile Phe Phe Ala Glu Ala Lys Lys Asn Gly Lys Lys
           30                  35                  40
   gaa ttc ttt ctt ttt tta cta aat ata aaa aaa aat agc caa cag aaa    375
   Glu Phe Phe Leu Phe Leu Leu Asn Ile Lys Lys Asn Ser Gln Gln Lys
       45                  50                  55
   aaa act tat cat att acc aaa agg aat acc ata aat aaa agt gat ttt    423
   Lys Thr Tyr His Ile Thr Lys Arg Asn Thr Ile Asn Lys Ser Asp Phe
   60                  65                  70                  75
   tta tat tct tta cta aat gaa gaa ggg aat tct tca aaa aag gaa tat    471
   Leu Tyr Ser Leu Leu Asn Glu Glu Gly Asn Ser Ser Lys Lys Glu Tyr
                   80                  85                  90
   aaa aat tta aaa gat gaa gaa aaa tat aat atc ata caa aat ata aaa    519
   Lys Asn Leu Lys Asp Glu Glu Lys Tyr Asn Ile Ile Gln Asn Ile Lys
               95                  100                 105
   aaa tat tgt gaa tgt act aaa aaa tat aaa agg ctc cca aca cga gaa    567
   Lys Tyr Cys Glu Cys Thr Lys Lys Tyr Lys Arg Leu Pro Thr Arg Glu
           110                 115                 120
   gta gtt att gga aat gtt aaa att gga gga aat aat aaa ata gct att    615
   Val Val Ile Gly Asn Val Lys Ile Gly Gly Asn Asn Lys Ile Ala Ile
   125 130 135
   caa act atg gct agc tgt gat aca aga aat gta gaa gaa tgt gta tat 663
   Gln Thr Met Ala Ser Cys Asp Thr Arg Asn Val Glu Glu Cys Val Tyr
   140 145 150 155
   caa att aga aaa tgt aaa gat ttg ggt gct gac att gta agg ttg act 711
   Gln Ile Arg Lys Cys Lys Asp Leu Gly Ala Asp Ile Val Arg Leu Thr
   160 165 170
   gtt caa gga gtt caa gaa gca caa gct agt tat cat att aaa gaa aaa 759
   Val Gln Gly Val Gln Glu Ala Gln Ala Ser Tyr His Ile Lys Glu Lys
   175 180 185
   tta tta tct gaa aat gta aat atc cca tta gta gca gat att cat ttt 807
   Leu Leu Ser Glu Asn Val Asn Ile Pro Leu Val Ala Asp Ile His Phe
   190 195 200
   aat cct aaa ata gct tta atg gca gct gat gtg ttt gaa aaa att cga 855
   Asn Pro Lys Ile Ala Leu Met Ala Ala Asp Val Phe Glu Lys Ile Arg
   205 210 215
   gtg aat cca gga aat tat gtt gat gga aga aaa aaa tgg ata gat aaa 903
   Val Asn Pro Gly Asn Tyr Val Asp Gly Arg Lys Lys Trp Ile Asp Lys
   220 225 230 235
   gtt tat aaa act aaa gaa gaa ttt gat gaa ggg aaa tta ttt ata aaa 951
   Val Tyr Lys Thr Lys Glu Glu Phe Asp Glu Gly Lys Leu Phe Ile Lys
   240 245 250
   gaa aaa ttt gta cca tta att gaa aaa tgt aaa aga tta aat aga gca 999
   Glu Lys Phe Val Pro Leu Ile Glu Lys Cys Lys Arg Leu Asn Arg Ala
   255 260 265
   ata aga att gga aca aat cat gga tcc ctt tca tct cga gta tta tca 1047
   Ile Arg Ile Gly Thr Asn His Gly Ser Leu Ser Ser Arg Val Leu Ser
   270 275 280
   tat tat gga gat aca cca tta ggt atg gta gaa tcg gct ttt gag ttt 1095
   Tyr Tyr Gly Asp Thr Pro Leu Gly Met Val Glu Ser Ala Phe Glu Phe
   285 290 295
   tct gat tta tgt att gaa aac aat ttt tac aat ctt gtt ttt tct atg 1143
   Ser Asp Leu Cys Ile Glu Asn Asn Phe Tyr Asn Leu Val Phe Ser Met
   300 305 310 315
   aaa gct tct aat gct tat gtt atg ata caa tct tat aga tta tta gta 1191
   Lys Ala Ser Asn Ala Tyr Val Met Ile Gln Ser Tyr Arg Leu Leu Val
   320 325 330
   tct aaa caa tat gaa aga aat atg atg ttc cct ata cat tta gga gtt 1239
   Ser Lys Gln Tyr Glu Arg Asn Met Met Phe Pro Ile His Leu Gly Val
   335 340 345
   aca gaa gca gga ttt ggt gat aat gga aga ata aaa tct tat tta ggt 1287
   Thr Glu Ala Gly Phe Gly Asp Asn Gly Arg Ile Lys Ser Tyr Leu Gly
   350 355 360
   ata gga tct tta tta tat gat ggt ata gga gat acc att cgt ata tcc 1335
   Ile Gly Ser Leu Leu Tyr Asp Gly Ile Gly Asp Thr Ile Arg Ile Ser
   365 370 375
   tta aca gaa gat cct tgg gaa gag tta act cct tgt aaa aaa tta gtt 1383
   Leu Thr Glu Asp Pro Trp Glu Glu Leu Thr Pro Cys Lys Lys Leu Val
   380 385 390 395
   gaa aat tta aag aaa aga ata ttt tat aat gaa aat ttt aaa gaa gat 1431
   Glu Asn Leu Lys Lys Arg Ile Phe Tyr Asn Glu Asn Phe Lys Glu Asp
   400 405 410
   aat gaa tta aaa aat aat gaa atg gat acc aaa aat cta tta aat ttt 1479
   Asn Glu Leu Lys Asn Asn Glu Met Asp Thr Lys Asn Leu Leu Asn Phe
   415 420 425
   gaa gaa aat tat cga aat ttt aat aat ata aaa aaa aga aat gta gaa 1527
   Glu Glu Asn Tyr Arg Asn Phe Asn Asn Ile Lys Lys Arg Asn Val Glu
   430 435 440
   aaa aat aat aat gta tta cat gaa gag tgc act ata ggt aat gta gta 1575
   Lys Asn Asn Asn Val Leu His Glu Glu Cys Thr Ile Gly Asn Val Val
   445 450 455
   acc ata aaa gag tta gaa gat tct ctg caa att ttt aaa gat tta aat 1623
   Thr Ile Lys Glu Leu Glu Asp Ser Leu Gln Ile Phe Lys Asp Leu Asn
   460                 465                 470                 475
   tta gaa gta gat tca aat gga aat ttg aaa aag gga gcc aaa aca act    1671
   Leu Glu Val Asp Ser Asn Gly Asn Leu Lys Lys Gly Ala Lys Thr Thr
                   480                 485                 490
   gat atg gtt att ata aat gat ttt cat aat ata aca aat tta gga aaa    1719
   Asp Met Val Ile Ile Asn Asp Phe His Asn Ile Thr Asn Leu Gly Lys
               495                 500                 505
   aaa act gtg gat aaa tta atg caa gtg gga att aat ata gta gtt caa    1767
   Lys Thr Val Asp Lys Leu Met Gln Val Gly Ile Asn Ile Val Val Gln
           510                 515                 520
   tat gaa cca cat aat ata gaa ttt ata gaa aaa atg gaa cca aat aat    1815
   Tyr Glu Pro His Asn Ile Glu Phe Ile Glu Lys Met Glu Pro Asn Asn
       525                 530                 535
   gat aat aat aat aat aat aat aat aat aat ata tta ttt tat gtg gat    1863
   Asp Asn Asn Asn Asn Asn Asn Asn Asn Asn Ile Leu Phe Tyr Val Asp
   540                 545                 550                 555
   ata aaa aat att atg aac agt tca gaa aaa aat att aaa tta agt aat    1911
   Ile Lys Asn Ile Met Asn Ser Ser Glu Lys Asn Ile Lys Leu Ser Asn
                   560                 565                 570
   tct aaa gga tat gga tta att tta aac gga aaa gaa gat ata caa acc    1959
   Ser Lys Gly Tyr Gly Leu Ile Leu Asn Gly Lys Glu Asp Ile Gln Thr
               575                 580                 585
   ata aaa aaa ata aaa gaa tta aat cgt cgt cct tta ttc att cta tta    2007
   Ile Lys Lys Ile Lys Glu Leu Asn Arg Arg Pro Leu Phe Ile Leu Leu
           590                 595                 600
   aaa tca gat aac ata tat gaa cat gta tta ata acc aga aga att aat    2055
   Lys Ser Asp Asn Ile Tyr Glu His Val Leu Ile Thr Arg Arg Ile Asn
       605                 610                 615
   gaa ctt tta caa tcc tta aat ata aat ata cct tat ata cat tat gtt    2103
   Glu Leu Leu Gln Ser Leu Asn Ile Asn Ile Pro Tyr Ile His Tyr Val
   620                 625                 630                 635
   gat att aat tca aac aat tat gat gat ata tta gtt aat tca aca tta    2151
   Asp Ile Asn Ser Asn Asn Tyr Asp Asp Ile Leu Val Asn Ser Thr Leu
                   640                 645                 650
   tat gca gga agt tgt ttg atg gat tta atg ggg gat ggt ctt att gtt    2199
   Tyr Ala Gly Ser Cys Leu Met Asp Leu Met Gly Asp Gly Leu Ile Val
               655                 660                 665
   aac gta act aat gat gtt ctt aca aat aaa aaa aag ata gaa aca aaa    2247
   Asn Val Thr Asn Asp Val Leu Thr Asn Lys Lys Lys Ile Glu Thr Lys
           670                 675                 680
   tat gat gaa aaa gaa gaa gta gag gaa gag gga aac aat aaa gat att    2295
   Tyr Asp Glu Lys Glu Glu Val Glu Glu Glu Gly Asn Asn Lys Asp Ile
       685                 690                 695
   cat aga ctt ttg agc aga gtt gca tta aat tca ttt tta aca tta aat    2343
   His Arg Leu Leu Ser Arg Val Ala Leu Asn Ser Phe Leu Thr Leu Asn
   700                 705                 710                 715
   att tta caa gat aca aga ata cgt tta ttt aaa aca gat tat ata gcc    2391
   Ile Leu Gln Asp Thr Arg Ile Arg Leu Phe Lys Thr Asp Tyr Ile Ala
                   720                 725                 730
   tgc cca tct tgt gga aga act tta ttt aat ata caa gaa act act aaa    2439
   Cys Pro Ser Cys Gly Arg Thr Leu Phe Asn Ile Gln Glu Thr Thr Lys
               735                 740                 745
   aaa att atg aaa tta aca ggg cac tta aaa ggc gtt aaa att gca gtc    2487
   Lys Ile Met Lys Leu Thr Gly His Leu Lys Gly Val Lys Ile Ala Val
           750                 755                 760
   atg gga tgt att gtt aat ggt ata gga gaa atg gca gat gca cat ttt    2535
   Met Gly Cys Ile Val Asn Gly Ile Gly Glu Met Ala Asp Ala His Phe
       765                 770                 775
   ggt tat gtt ggt agt gca cct aaa aaa att gat tta tat tat ggt aaa    2583
   Gly Tyr Val Gly Ser Ala Pro Lys Lys Ile Asp Leu Tyr Tyr Gly Lys
   780                 785                 790                 795
   gag tta gta gaa aga aat ata cct gag gaa gaa gct tgt gat aaa ttg    2631
   Glu Leu Val Glu Arg Asn Ile Pro Glu Glu Glu Ala Cys Asp Lys Leu
   800 805 810
   ata gaa tta att aaa aaa cat aac aaa tgg aaa gat cca taaattgaat 2680
   Ile Glu Leu Ile Lys Lys His Asn Lys Trp Lys Asp Pro
   815 820
   atggacaagt atttatttat ttatttatct tatatataat atattataaa tttttcgatg 2740
   tatttttcct tttaaaattt tatttttttt ttattttttt ttttgaagta atatttataa 2800
   tgcatacata atattaaaat gtgtattata taataatatc attttattgt tattttaaaa 2860
   gactaatacc aagaacaatt ttttaataat cattcttata acttgttaaa tatatatata 2920
   tatatatata tatttattta tttatattta tatttattta tttttggtat atgaaaagta 2980
   aaaatataat aatttaaaag tatttacaaa ataaataata ttatatatct gtttttatat 3040
   atatgttaat ggaaaaggag aaaataaata aataaaacaa acaaaataac atatatatat 3100
   atatatatat actgaatgag aaagaaaaaa aaaagaaaag gatacga 3147
<210> 6
   <211> 824
   <212> PRT
   <213> Plasmodium falciparum
<400> 6

## Beschreibung

Die vorliegende Erfindung betrifft DNA-Sequenzen, die bei Integration in das Genom von Viren, Eukaryonten und Prokaryonten die Isoprenoid-Biosynthese verändern sowie gantechnologische Verfahren zur Herstellung dieser transgenen Viren, Eukaryonten und Prokaryonten. Außerdem betrifft sie Verfahren zur Identifiziereung von Stoffen mit herbizider, antimikrobieller, antiparasitärer, antiviraler, fungizider, bakterizider Wirkung bei Pflanzen und antimikrobieller, antiparasitärer, antimykotischer, antibakterieller und antiviraler Wirkung bei Mensch und Tier.

Der Biosyntheseweg zur Bildung von Isoprenoiden über den klassischen Acetat/ Mevalonat-Weg und einen alternativen, Mevalonat-unabhängigen Biosyntheseweg, den Desoxy-D-xylulose-Phosphat-Weg, ist bereits bekannt (Rohmer, M., Knani, M., Simonin, P., Sutter, B., and Sahm, H. (1993): Biochem. J. 255: 517-524) .

Im Stand der Technik (Lange et al "A family of transketolases that directs isoprenoid biosynthesis via a mevalonate-independent pathway" EASEB JOURNAL, US, FED. OF AMERICAN SOC. FOR EXPERIMENTAL BIOLOGY, BETHESDA, MD", Kuzuyama et al: "Direct formation of 2-C-Methyl-D-Erythritol 4-phosphate from 1-Deoxy-D-Xylulose 5-phosphate Reductoisomerase, a new enzyme in the non-mevalonate pathway to isopentenyl diphosphate" TETRAHEDRON LETTERS, NL, ELSEVIER SCIENCE PUBLISHERS, AMSTERDAM", Putra et al "Incorporation of [2,3-[13]C2]- and [2,4-[13]C2]-D-1-Deoxyxylulose into ubiquinone of Escherichia coli via the Mevalonate-Independent pathway for Isoprenoid Biosynthesis" TETRAHEDRON LETTERS, NL, ELSEVIER SCIENCE PUBLISHERS, AMSTERDAM") ist bekannt, dass DOXP Synthase, DOXP Reductoisomerase und gcpE als Gene aus Pfefferminze und E.coli isoliert wurden. Ferner geht der Stand der Technik davon aus, dass der DOXE-Stoffwechselweg nur in Bakterien und Pflanzen vorkommt (Lange et al (supra), letzter Absatz).

Zudem ist nicht bekannt, wie und über welche Wege in Viren, Eukaryonten und Prokaryonten eine Änderung der Isoprenoidkonzentratian über den Desoxy-D-xylulose-Phoshat-Weg erreicht werden kann. In Fig. 1 ist dieser Biosyntheseweg dargestellt.

Es werden daher DNA-Sequenzen zur Verfügung gestellt, die für die 1-Desoxy-D-xylulose-5-phosphat-Synthase (DOXP-Synthase), : 1-Desoxy-D-xylulose-5-phosphatreduktoisomerase (DOXF-Reduktoisonterase) oder das gcpE-Protein kodieren. Alle drei Gene und Enzyme sind an der Isoprenoid-Biosynthese beteiligt.

Das gcpE-Protein hat eine Kinasefunktion und katalysiert die Phosphorylierung eines Zuckers oder eines Phosphorzuckers oder einer Vorstufe der Isoprenoidbiosynthese, insbesondere die Phosphorylierung von 2-C-Methyl-D-erythritol, 2-C-Mechyl-D-erythritol-phosphat, insbesondere 2-C-Methyl-D-erythritol-4-phosphat, 2-C-Methyl-D-erythrose, 2-C-Methyl-D-erythrose-phosphat, insbesondere 2-C-Methyl-D-erythrose-4-phosphat. In der Vorstufe der Isoprenoidsynthese katalysiert das gcpE-Protein insbesondere die Phosphorylierung der folgenden Substanzen:
CH₂ (OH) -C (CH₃) =C (OH) -CH₂-O-PO (OH)₂, CH₂ (OH) -C (CH₃) =C (OH) -CH₂-OH,
CH₂(OH)-CH(CH₃)-CO-CH₂-O-PO(OH)₂, CH₂(OH)-CH(CH₃)-CO-CH₂-OH
CK₂=C (CH₃) -CO-CH₂-O-PO (OH) ₂, CH₂=C (CH₃) -CO-CH₂-OH,
CH₂=C(CH₃) -CH(OH) -CH₂-O-PO(OH)₂, CH₂=C(CH₃) -CH(OH) -CH₂-OH,
CH₂ (OH) -C (=CH₂) -C (OH) -CH₂-O-PO (OH) ₂, CH₂ (OH) -C (=CH₂) -C (OH) -CH₂-OH
CHO-CH(CH₃)-CH(OH)-CH₂-O-PO(OH)₂, CHO-CH(CH₃)-CH(OH)-CH₂-OH,
CH₂ (OH) -C (OH) (CH₃) -CH=CH-O-PO (OH)₂, CH₂ (OH) -C (OH) (CH₃) -CH=CH-OH
CH(OH)=C(CH₃)-CH(OH)-CH₂-O-PO(OH)₂, CH(OH)=C(CH₃)-CH(OH)-CH₂-OH,
(CH₃)₂HC-CO-CH₂-O-PO(OH)₂, (CH₃)₂HC-CO-CH₂-O-H,
(CH₃)₂HC-CH (OH) -CH₂-O-PO (OH) ₂, (CH₃)₂HC-CH (OH) -CH₂-O-H.

Die DOXP-Synthase katalysiert die Kondensation von Pyruvat und Glyceraldehyd-3-phosphat zu 1-Deoxy-D-xylulose-5-phosphat und die DOXP-Reduktoisomerase katalysiert die Umwandlung von 1-Deoxy-D-xylulose-5-phosphat zu 2-C-Methyl-D-erythritol-4-phosphat.

Die Erfindung betrifft die DNA- und Protein-Sequenzen gemäß den Ansprüchen 1 bis 2 und 8 und insbesondere DNA-Sequenzen, die für ein Polypeptid mit der in SEQ ID NO: 6 dargestellten Aminosäuresequenz codieren oder für ein Analoges oder Derivat des Polypeptids gemäß SEQ ID NO: 6, worin eine oder mehrere Aminosäuren deletiert, hinzugefügt oder durch andere Aminosäuren substituiert worden sind, wobei die katalytische Funktion des Polypeptids erhalten bleibt.

Die Gene und ihre Genprodukte (Polypeptide) sind im Sequenzprotokoll mit ihrer Primärstruktur aufgeführt und haben folgende Zuordnung :
SEQ ID NO:1: 1-Desoxy-D-xylulose-5-phosphatreduktoisomerase-Gen
SEQ ID NO:2: 1-Desoxy-D-xylulose-5-phosphatreduktoisomerase
SEQ ID NO:3: 1-Desoxy-D-xylulose-5-phosphat-Synthase-Gen
SEQ ID NO:4: 1-Desoxy-D-xylulose-5-phosphat-Synthase
SEQ ID NO:5: gcpE-Gen
SEQ ID NO:6 : gcpE-Proteine.

Die DNA-Sequenzen stammen alle aus Plasmodium falciparum.

Außer den im Sequenzprotokoll genannten DNA-Sequenzen sind auch solche geeignet, die infolge der Degeneration des genetischen Codes eine andere DNA-Sequenz besitzen, jedoch für das gleiche Polypeptid.

Die erfindungsgemäßen Sequenzen eignen sich für die Expression von Genen in Viren, Eukaryonten und Prokaryonten, die für die Isoprenoid-Biosynthese des 1-Desoxy-D-xylulose-Wegs verantwortlich sind.

Erfindungsgemäß gehören zu den Eukaryonten oder eukaryontischen Zellen tierischen Zellen, Pflanzenzellen, Algen, Hefen, Pilze und zu den Prokaryonten oder prokaryontischen Bakterien Archaebakterien und Eubakterien.

Bei Integration einer DNA-Sequenz in ein Genom, auf der eine der oben angegebenen DNA-Sequenzen lokalisiert ist, wird die Expression der oben beschriebenen Gene in Viren, Eukaryonten und Prokaryonten ermöglicht. Die erfindungsgemäß transformierten Viren, Eukaryonten und Prokaryonten werden in an sich bekannter Weise gezüchtet und das währenddessen gebildete Isoprenoid isoliert und gegebenenfalls gereinigt. Nicht alle Isoprenoide müssen isoliert werden, da die Isoprenoide in einigen Fällen direkt in die Raumluft abgegeben werden.

Die Erfindung betrifft ferner ein Verfahren zur Herstellung von transgenen Viren, Eukaryonten und Prokaryonten zur Veränderung des Isoprenoid-Gehaltes, das die folgenden Schritte enthält.
a) Herstellung einer DNA-Sequenz mit folgenden Teilsequenzen
   i) Promotor, der in Viren, Eukaryonten und Prokaryonten aktiv ist und die Bildung einer RNA im vorgesehenen Zielgewebe oder den Zielzellen sicherstellt,
   ii) DNA-Sequenz, das für ein Polypeptid mit der in SEO ID NO: 6 dargestellten Aminosäuresequenz codiert oder für ein Analoges oder Derivat des Polypeptids gemäß SEQ ID NO: 6,
   iii) 5'- und 3'-nichttranslatierte Sequenz, die in Viren, Eukaryonten und Prokaryonten die Expression der bezeichneten Gene ermöglichen oder verbessern,
b) Transfer und Einbau der DNA-Sequenz in das Genom von Viren, prokaryontischen oder eukaryontischen Zellen mit oder ohne Verwendung eines Vektors (z.B. Plasmid, virale DNA).

Aus derart transformierten Pflanzenzellen können die intakten ganzen Pflanzen regeneriert werden.

Die für das Protein codierende Sequenz mit der Nukleotidabfolgen Seq ID NO: 5 können mit einem die Transkription in bestimmten Organen oder Zellen sicherstellenden Promotor versehen werden, der in sense-Orientierung (3'-Ende des Promotors zum 5'-Ende der kodierenden Sequenz) an die Sequenz, die das zu bildende Protein kodiert, gekoppelt ist. An das 3'-Ende der kodierenden Seqeunz wird ein die Termination der mRNA-Synthese bestimmendes Terminationssignal angehängt. Um das zu exprimierende Protein in bestimmte subzelluläre Kompartimente, wie Chloroplasten, Amyloplasten, Mitochondrien, Vakuole, Cytosol oder Interzellularräume zu dirigieren, kann zwischen den Promotor und die kodierende Sequenz noch eine für eine sogenannte Signalsequenz oder ein Transitpeptid kodierende Sequenz gesetzt werden. In einigen Fällen ist es erforderlich, Sequenzen einzufügen, die für eine Signalsequenz am COOH-Terminus des Proteins kodieren. Die Sequenz muß im gleichen Leserahmen wie die kodierende Sequenz des Proteins sein. Zur Vorbereitung der Einführung der erfindungsgemäßen DNA-Sequenzen in höhere Pflanzen sind eine große Anzahl von Klonierungsvektoren verfügbar, die ein Replikationssignal für E. coli und einen Marker beinhalten, der eine Selektion der transformierten Zellen erlaubt. Je nach Einführungsmethode gewünschter Gene in die Pflanze können weitere DNA-Sequenzen erforderlich sein. Werden zum Beispiel für die Transformation der Pflanzenzelle das Ti- oder Ri-Plasmid verwendet, so muß mindestens eine rechte Begrenzung, häufig jedoch die rechte und die linke Begrenzung der Ti- und Ri-Plasmid T-DNA als Flankenbereich den einzuführenden Genen eingefügt werden. Die Verwendung von T-DNA für die Transformation von Pflanzenzellen ist intensiv untersucht und ausreichend in EP 120516; Hoekama, in: The Binary Plant Vector System, Offset-drukkerij Kanters B.V. Alblasserdam (1985), Chapter V; Fraley et al., Crit.Rev.Plant Sci. 4,1-46 und An et al. (1985) EMBO J. 4, 277-287 beschrieben worden. Ist die eingeführte DNA einmal im Genom integriert, so ist sie in der Regel stabil und bleibt auch in den Nachkommen der ursprünglich transformierten Zellen erhalten. Sie erhält normalerweise einen Selektionsmarker, der den transformierten Pflanzenzellen Resistenz gegenüber einem Biozid oder einem Antibiotikum, wie Kanamycin, G 418, Bleomycin, Hygromycin oder Phosphinotricin u.a. vermittelt. Der individuell verwendete Marker sollte daher die Selektion transformierter Zellen gegenüber Zellen, denen die eingefügte DNA fehlt, gestatten.

Für die Einführung von DNA in eine Pflanze stehen viele Techniken zur Verfügung. Diese Techniken umfassen die Transformation mit Hilfe von Agrobakterien, z.B. Agrobacterium tumefaciens die Fusion von Protoplasten, die Mikroinjektion von DNA, die Elekroporation, sowie ballistische Methoden und die Virusinfektion. Aus dem transformierten Pflanzenmaterial können dann im geeigneten Medium, welches Antibiotika oder Biozide zur Selektion enthalten kann, wieder ganze Pflanzen regeneriert werden. Bei der Injektion und Elektroporation sind an sich keine speziellen Anforderungen an die Plasmide gestellt. Sollen aber aus derartig transformierten Zellen ganze Pflanzen regeneriert werden, ist die Anwesenheit eines selektierbaren Markergens notwendig. Die transformierten Zellen wachsen innerhalb der Pflanzen in der üblichen Weise (McCormick et al. (1986), Plant Cell Reports 5, 81-84). Die Pflanzen können normal angezogen werden und mit Pflanzen, die die gleiche transformierte Erbanlage oder andere Erbanlagen haben, gekreuzt werden. Die daraus entstehenden Individuen haben die entsprechenden phänotypischen Eigenschaften.

Weiterhin sind Gegenstand der Erfindung Expressionsvektoren, die eine oder mehrere der erfindungsgemäßen DNA-Sequenzen enthalten. Solche Expressionsvektoren erhält man, indem man die erfindungsgemäßen DNA-Sequenzen mit geeigneten funktionellen Regulationssignalen versieht. Solche Regulationssignale sind DNA-Sequenzen, die für die Expression verantwortlich sind, beispielsweise Promotoren, Operatoren, Enhancer, ribosomale Bindungsstellen, und die vom Wirtsorganismus erkannt werden.

Gegebenenfalls können noch weitere Regulationssignale, die beispielsweise Replikation oder Rekombination der rekombinanten DNA im Wirtsorganismus steuern, Bestandteil des Expressionsvektors sein.

Ebenso gehören die mit den erfindungsgemäßen DNA-Sequenzen oder Expressionsvektoren transformierten Wirtsorganismen zum Gegenstand der Erfindung.

Für die Expression der erfindungsgemäßen Enzyme eignen sich besonders solche Wirtszellen und Organismen, die keine intrinsischen Enzyme mit der Funktion der DOXP-Synthase, der DOXP-Reduktoisomerase oder des gcpE-Proteins aufweisen. Dies trifft für Archaebacterien, Tiere, Pilze, Schleimpilze und einige Eubakterien zu. Durch das Fehlen dieser intrinsischen Enzymaktivitäten wird die Detektion und Aufreinigung der rekombinanten Enzyme wesentlich erleichtert. Auch wird es erst dadurch möglich, mit geringem Aufwand die Aktivität und insbesondere die Hemmung der Aktivität der erfindungsgemäßen rekombinanten Enzyme durch verschiedenen Chemikalien und Pharmaka in Rohextrakten aus den Wirtszellen zu messen.

Die Expression der erfindungsgemäßen Enzyme erfolgt vorteilhafterweise dann in eukaryontischen Zellen, wenn posttranslatorische Modifikationen und eine native Faltung der Polypeptidkette erreicht werden soll. Außerdem wird in Abhängigkeit vom Expressionssystem bei der Expression genomischer DNA-Sequenzen erreicht, daß Introns durch Spleißen der DNA beseitigt und die Enzyme in der für die Parasiten charakteristischen Polypeptidsequenz produziert werden. Für Introns codierende Sequenzen können auch durch rekombinante DNA-Technologie aus den zu exprimierenden DNA-Sequenzen beseitigt oder experimentell eingefügt werden.

Die Isolierung des Proteins kann aus der Wirtszelle oder dem Kulturüberstand der Wirtszelle nach dem Fachmann bekannten Verfahren erfolgen. Es kann auch eine in vitro Reaktivierung der Enzyme erforderlich sein.

Zur Erleichterung der Aufreinigung können die erfindungsgemäßen Enzyme oder Teilsequenzen der Enzyme als Fusionsprotein mit verschiedenen Peptidketten exprimiert werden. Dazu eigenen sich besonders Oligo-Histidin-Sequenzen und Sequenzen, die von der Glutathion-S-Transferase, Thioredoxin oder Calmodulin-bindenden Peptiden abgeleitet sind.

Weiterhin können die erfindungsgemäßen Enzyme oder Teilsequenzen der Enzyme als Fusionsprotein mit solchen, dem Fachmann bekannten, Peptidketten exprimiert werden, daß die rekombinanten Enzyme in das extrazelluläre Millieu oder in bestimmte Kompartimente der Wirtszellen transportiert werden. Dadurch kann sowohl die Aufreinigung, als auch die Untersuchung der biologischen Aktivität der Enzyme erleichtert werden.

Bei der Expression der erfindungsgemäßen Enzyme kann es sich als zweckmäßig erweisen, einzelne Codone zu verändern. Dabei ist der gezielte Austausch von Basen in der kodierenden Region auch sinnvoll, wenn die genutzten Codone in den Parasiten abweichend sind von der Codonnutzung im heterologen Expressionssystem, um eine optimale Synthese des Proteins zu gewährleisten.

Weiterhin können die erfindungsgemäßen Enzyme unter standardisierten Bedingungen durch dem Fachmann bekannte Techniken durch in vitro-Translation gewonnen werden. Dafür geeignete Systeme sind Kaninchen-Reticulozyten- und Weizenkeimextrakte und Bakterienlysate. Auch kann in vitro transskribierte mRNA in Xenopus-Oocyzen translatiert werden.

Durch chemische Synthese können Oligo- und Polypeptide hergestellt werden, deren Sequenzen aus der Peptidsequenz der erfindungsgemäßen Enzyme abgeleitet sind. Bei geeigneter Wahl der Sequenzen besitzen derartige Peptide Eigenschaften, die für die erfindungsgemäßen Enzyme charakteristisch sind. Derartige Peptide können in großen Mengen hergestellt werden und eignen sich besonders für Studien über die Kinetik der Enzymaktivität, die Regulation der Enzymaktivität, die dreidimensionale Struktur der Enzyme, die Hemmung der Enzymaktivität durch verschiedenen Chemikalien und Pharmaka und die Bindungsgeometrie und Bindugnsaffinität verschiedener Liganden.

Vorzugsweise wird zur rekombinanten Herstellung des erfindungsgemäßen Enzyms eine DNA mit den Nukleotiden aus der Sequenz SEQ ID NO: 5 verwendet.

Die Erfindung umfasst daher außerdem ein Verfahren zum Screening nach Verbindungen, die den Desoxy-D-xylulose-Phosphat-Stoffwechselweg inhibieren. Gemäß diesem Verfahren wird ein Wirtsorganismus, der einen rekombinanten Expressionsvektor enthält, wobei der Vektor zumindest einen Teil der Olignukleotidsequenz gemäß SEQ ID NO: 5 oder Varianten oder Homologe dieser aufweist, und außerdem eine Verbindung, von der vermutet wird, daß sie eine antimikrobielle, antiparasitäre, antibakterielle, antivirale und antimykotische Wirkung bei Mensch und Tier oder eine antimikrobielle, antivirale, bakterizide, herbizide oder fungizide Wirkung bei Pflanzen hat, bereitgestellt. Anschließend wird der Wirtsorganismus mit der Verbindung in Kontakt gebracht und die Wirksamkeit der Verbindung bestimmt.

Ein weiterer Gegenstand dieser Erfindung sind Methoden zur Bestimmung der enzymatische Aktivität des gcpE-Proteins. Diese kann nach bekannten Verfahren bestimmt werden. Hierbei wird die Phosphorylierung eines Zuckers oder eines Phosphorzuckers oder einer Vorstufe der Isoprenoidbiosynthese, insbesondere die Phosphorylierung von 2-C-Methyl-D-erythritol, 2-C-Methyl-D-erythritol-phosphat, insbesondere 2-C-Methyl-D-erythritol-4-phosphat, 2-C-Methyl-D-erythrose, 2-C-Methyl-D-erythrose-phosphat, insbesondere 2-C-Methyl-D-erythrose-4-phosphat, detektiert. Ein weiterer Gegenstand dieser Erfindung ist die Verwendung dieser Meßverfahren zur Ermittlung von Stoffen, die die Aktivität der jeweiligen Enzyme inhibieren.

Die enzymatische Aktivität von DOXP-Synthase und DOXP-Reduktisomerase kann in einem einzigen Schitt detektiert werden, indem die Umwandlung von Glycerinaldehyd-3-phosphat zu 2-C-Methylerythritol-4-phosphat bestimmt wird.

Analog erfolgt die Bestimmung der Aktivitäten von DOXP-Synthase und DOXP-Reduktoisomerase. Für die Bestimmung der DOXP-Synthase-Aktivität eignen sich auch fluorimetrische Verfahren, wie von Querol et al. beschrieben (Querol et al. Abstracts 4^{th} european symposium on plant isoprenoids, Barcelona 21-23 April 1999).

## Patentansprüche

1. DNA-Sequenzen codierend für ein Polypeptid mit der in SEQ ID NO: 6 dargestellten Aminosäuresequenz.

2. DNA-Sequenz gemäß SEQ ID NO: 5 codierend für ein Polypeptid mit der in SEQ ID NO: 6 dargestellten Aminosäuresequenz.

3. DNA-Sequenz gemäß Anspruch 1 oder 2, **dadurch gekennzeichnet, daß** diese außerdem funktionelle Regulationssignale, insbesondere Promotoren, Operatoren, Enhancer, ribosomale Bindungsstellen, aufweist.

4. DNA-Sequenz gemäß einem der Ansprüche 1 bis 3, mit folgenden Teilsequenzen
i) Promotor, der in Viren, Eukaryonten und Prokaryonten aktiv ist und die Bildung einer RNA im vorgesehenen Zielgewebe oder den Zielzellen sicherstellt,
ii) DNA-Sequenzen gemäß einem der Ansprüche 1 bis 3,
iii) 3'-nichttranslatierte Sequenz, die in Viren, Eukaryonten und Prokaryonten zur Addition von Poly-A Resten an das 3'-Ende der RNA führt.

5. Verfahren zur Herstellung von transgenen Viren, Eukaryonten, wobei humane Eukaryonten ausgenommen sind, und Prokaryonten zur Veränderung des Isoprenoid-Gehaltes, **dadurch gekennzeichnet, daß** eine DNA-Sequenz gemäß Anspruch 3 oder 4 in das Genom von Viren, eukaryontischen und prokaryontischen Zellen mit oder ohne Verwendung eines Plamids transferiert und eingebaut wird.

6. Transgene Systeme, insbesondere Pflanzen und Pflanzenzellen, wobei humane transgene Systeme ausgenommen sind, welche eine DNA-Sequenz nach einem der Ansprüche 1 bis 3 enthält, welches exprimiert wird.

7. Expressionsvektor, enthaltend eine oder mehrere DNA-Sequenzen gemäß einem der Ansprüche 1 bis 4.

8. Protein, welches am 1-Deoxy-D-Xylulose-5-Phosphat-Stoffwechselweges beteiligt ist und codiert wird von einer DNA-Sequenz SEQ ID NO: 5.

9. Protein nach Anspruch 8, erhältlich aus den Kulturüberständen von Parasiten oder aus den aufgeschlossenen Parasiten und Aufreinigung über chromatographische und elektrophoretische Techniken.

10. Protein nach einem der Ansprüche 8 oder 9, **dadurch gekennzeichnet, daß** es das Produkt einer viralen, prokaryontischen oder eukaryontischen Expression einer exogenen DNA ist.

11. Protein gemäß einem der vorangehenden Ansprüchen, welches aus einer Aminosäuresequenz gemäß SEQ ID NO: 6 besteht.

12. Verfahren zur Bestimmung der enzymatische Aktivität des gcpE-Proteins, **dadurch gekennzeichnet, daß** Phosphorylierung eines Zuckers oder eines Phosphorzuckers oder einer Vorstufe der Isoprenoidbiosynthese, insbesondere die Phosphorylierung von 2-C-Methyl-D-erythritol, 2-C-Methyl-D-erythritol-phosphat, insbesondere 2-C-Methyl-D-erythritol-4-phosphat, 2-C-Methyl-D-erythrose, 2-C-Methyl-D-erythrose-phosphat, insbesondere 2-C-Methyl-D-erythrose-4-phosphat, und der Phosphat- und Alkoholvorstufen, detektiert wird.

13. Verfahren nach Anspruch 12, **dadurch gekennzeichnet, dass** die Phosphorylierung der folgenden Phosphate oder Alkohole detektiert wird:
CH₂(OH)-C(CH₃)=C(OH)-CH₂-O-PO(OH)₂,
CH₂ (OH) -C(CH₃) =C (OH) -CH₂-OH,
CH₂(OH)-CH(CH₃)-CO-CH₂-O-PO(OH)₂,
CH₂ (OH) -CH (CH₃) -CO-CH₂-OH,
CH₂=C(CH₃)-CO-CH₂-O-PO(OH)₂,
CH₂=C (CH₃) -CO-CH₂-OH,
CH₂=C(CH₃)-CH(OH)-CH₂-O-PO(OH)₂,
CH₂=C(CH₃)-CH(OH)-CH₂-OH,
CH₂(OH)-C(=CH₂)-C(OH)-CH₂-O-PO(OH)₂,
CH₂ (OH) -C (=CH₂) -C (OH) -CH₂-OH,
CHO-CH(CH₃)-CH(OH)-CH₂-O-PO(OH)₂,
CHO-CH(CH₃)-CH(OH)-CH₂-OH,
CH₂ (OH) -C (OH) (CH₃) -CH=CH-O-PO (OH)₂,
CH₂ (OH) -C (OH) (CH₃) -CH=CH-OH,
CH(OH)=C(CH₃)-CH(OH)-CH₂-O-PO(OH)₂,
CH(OH)=C(CH₃)-CH(OH)-CH₂-OH,
(CH₃)₂ CH-CO-CH₂-O-PO (OH)₂,
(CH₃)₂CH-CO-CH₂-OH,
(CH₃) ₂ CH-CH(OH)-CH₂-O-PO(OH)₂,
(CH₃) ₂ CH-CH (OH) -CH₂-OH.

## Claims

1. DNA sequences coding for a polypeptide having the amino acid sequence as set forth in SEQ ID NO: 6.

2. DNA sequence according to SEQ ID NO: 5 coding for a polypeptide having the amino acid sequence as set forth in SEQ ID NO: 6.

3. The DNA sequence according to claim 1 or 2, **characterized in that** said sequences also comprise functional regulatory signals, in particular promoters, operators, enhancers, ribosomal binding sites.

4. The DNA sequence according to one of the claims 1 to 3 having the following partial sequences
i) promoter, which is active in viruses, eukaryotes, and prokaryotes and which ensures the formation of RNA in the intended target tissue or the target cells,
ii) DNA sequences according to one of the claims 1 to 3,
iii) 3' untranslated sequence, which results in the addition of poly(A) residues at the 3' end of the RNA in viruses, eukaryotes, and prokaryotes.

5. Method for the preparation of transgenic viruses, eukaryotes, except from human eukaryotes, and prokaryotes for the modification of the concentration of isoprenoid, **characterized in that** a DNA sequence according to claim 4 or 5 is transferred and incorporated into the genome of viruses, eukaryotic and prokaryotic cells with or without the use of a plasmid.

6. Transgenic systems, in particular plants and plant cells, except from human transgenic systems, comprising one or more DNA sequences according to one of the claims 1 to 5, being expressed.

7. Expression vector comprising one or more DNA sequences according to claims 1 to 4

8. Protein involved in the 1-deoxy-D-xylulose-5-phosphate metabolism pathway and which is coded by the DNA sequence of SEQ ID NO: 5.

9. Protein according to claim 8 obtainable from the supernatant of cultures of parasites or from disrupted parasites and purified with chromatographic and electrophoretic techniques.

10. Protein according to one of the claims 8 or 9, **characterized in that** it is the product of a viral, prokaryotic or eukaryotic expression of an exogenic DNA.

11. Protein according to one of the preceding claims consisting of one of the amino acid sequence of SEQ ID NO: 6.

12. Method for determining the enzymatic activity of the gcpE protein, **characterized in that** the phosphorylation of a sugar or of a phosphorus sugar or of a precursor of the isoprenoid biosynthesis, in particular the phosphorylation of 2-C-methyl-D-erythritol, 2-C-methyl-D-erythritol phosphate, in particular 2-C-methyl-D-erythritol 4-phosphate, 2-C-methyl-D-erythrose, 2-C-methyl-D-erythrose phosphate, in particular 2-C-methyl-D-erythrose 4-phosphate, and of the phosphate and alcohol precursors is detected.

13. Method according to claim 12 **characterized in that** the phosphorylation of the following phosphates or alcohols is detected:
CH₂ (OH) -C (CH₃) = C (OH) -CH₂-O-PO (OH)₂,
CH₂ (OH) -C (CH₃) = C (OH) -CH₂-OH,
CH₂ (OH) -C (CH₃) -CO-CH₂-O-PO (OH)₂,
CH₂ (OH) -CH (CH₃) -CO-CH₂-OH,
CH₂ =C (CH₃) -CO-CH₂-O-PO (OH)₂,
CH₂ =C (CH₃) -CO-CH₂-OH,
CH₂=C (CH₃) -CH (OH)-CH₂-O-PO (OH)₂,
CH₂=C (CH₃) -CH (OH)-CH₂-OH,
CH₂ (OH) -C (=CH₂) -C (OH) -CH₂-O-PO (OH)₂,
CH₂ (OH) -C (=CH₂) -C (OH) -CH₂-OH,
CHO-CH (CH₃) -CH (OH) -CH₂-O-PO (OH)₂,
CHO-CH (CH₃) -CH (OH) -CH₂-OH,
CH₂ (OH) -C (OH) (CH₃)-CH=CH-O- PO (OH)₂,
CH₂ (OH) -C (OH) (CH₃) -CH=CH-OH,
CH (OH) =C (CH₃) -CH (OH) -CH₂-O-PO (OH)₂,
CH (OH) =C (CH₃) -CH (OH) -CH₂-OH,
(CH₃)₂ CH-CO-CH₂-O-PO (OH)₂,
(CH₃)₂ CH-CO-CH₂-OH,
(CH₃)₂ CH-CH (OH) -CH₂-O-PO (OH)₂,
(CH₃)₂ CH-CH (OH) -CH₂-OH.

## Revendications

1. Séquences ADN, codante pour un polypeptide comportant la séquence d'acides aminés présentée dans SEQ ID N° 6.

2. Séquence ADN correspondant à SEQ ID N° 5, codante pour un polypeptide comportant la séquence d'acides aminés présentée dans SEQ ID N° 6.

3. Séquence ADN selon revendications 1 ou 2,
**caractérisée en ce que** celle-ci présente des signaux régulateurs particulièrement fonctionnels, notamment des promoteurs, opérateurs, amplificateurs, sites de liaisons ribosomales.

4. Séquence ADN selon l'une quelconque des revendications 1 à 3, comportant les séquences partielles suivantes :
i) promoteur, qui est actif dans les virus, eucaryotes et procaryotes, et qui garantit la formation d'un ARN dans le tissu cible ou dans les cellules cibles prévues,
ii) des séquences ADN selon l'une quelconque des revendications 1 à 3,
iii) une séquence non traduite en 3' qui, dans les virus, eucaryotes et procaryotes, donne lieu à l'ajout de radicaux poly-A sur l'extrémité 3' de l'ARN.

5. Procédé de production de virus transgéniques, eucaryotes, les eucaryotes humains étant exclus, et de procaryotes permettant de modifier la teneur en isoprénoïdes, **caractérisé en ce qu'**une séquence ADN est transférée ou intégrée selon l'une quelconque des revendications 4 ou 5 dans le génome de virus, cellules eucaryotes et procaryotes en utilisant ou sans utiliser de plasmide.

6. Systèmes transgéniques, en particulier plantes et cellules de plantes, les systèmes transgéniques humains étant exclus, qui contiennent une ou plusieurs séquences ADN selon l'une quelconque ou plusieurs des revendications 1 à 5, qui sont exprimées.

7. Vecteur d'expression, contenant une ou plusieurs séquences ADN selon l'une quelconque des revendications 1 à 4.

8. Protéine, qui est fractionnée au niveau de la voie métabolique de 1-déoxy-D-xylulose-5-phosphate et est codée par une séquence ADN ID SEQ N° : 5.

9. Protéine selon la revendication 8, pouvant être obtenue à partir de surnageants de cultures de parasites ou des parasites isolés, et purification par l'intermédiaire de techniques de chromatographie et d'électrophorèse.

10. Protéine selon l'une quelconque des revendications 8 ou 9, **caractérisée en ce que** le produit est l'expression virale, procaryotique ou eucaryotique d'un ADN exogène.

11. Protéine selon l'une quelconque des revendications précédentes, qui est constituée d'une séquence d'acides aminés SEQ ID N° 6.

12. Procédé de détermination de l'activité enzymatique de la gcpE protéine, **caractérisé en ce que** la phosphorylation d'un sucre ou d'un phosphoglucose ou d'un progéniteur de la biosynthèse d'isoprénoïde, en particulier la phosphorylation du 2-C-méthyl-D-érythritol, 2-C-méthyl-D-érythritol-phosphate, en particulier 2-C-méthyl-D-érythritol-4-phosphate, 2-C-méthyl-D-érythrose, 2-C-méthyl-D-érythrose-phosphate, en particulier 2-C-méthyl-D-érythrose-4-phosphate, et des progéniteurs de phosphate et d'alcool, sont détectées.

13. Procédé selon la revendication 12, **caractérisé en ce que** la phosphorylation des phosphates ou alcools suivants est détectée :
CH₂ (OH) -C (CH₃) =C (OH) -CH₂-O-PO (OH)₂,
CH₂ (OH) -C (CH₃) =C (OH) -CH₂-OH),
CH₂(OH)-CH(CH₃)-CO-CH₂-O-PO(OH)₂,
CH₂(OH) -CH (CH₃) -CO-CH₂-OH,
CH₂=C (CH₃) -CO-CH₂-O-PO (OH)₂,
CH₂=C (CH₃)-CO-CH₂-OH,
CH₂=C (CH₃) -CH (OH) -CH₂-O-PO(OH)₂,
CH₂=C(CH₃)-CH(OH)-CH₂-OH,
CH₂(OH)-C(=CH₂)-C(OH)-CH₂-O-PO(OH)₂,
CH₂(OH)-C(=CH₂)-C(OH)-CH₂-OH,
CHO-CH(CH₃)-CH(OH)-CH₂-O-PO(OH)₂,
CHO-CH(CH₃)-CH(OH)-CH₂-OH,
CH₂ (OH) -C (OH) (CH₃)-CH=CH-O-PO(OH)₂,
CH₂ (OH)-C(OH) (CH₃) -CH=CH-OH,
CH(OH)=C(CH₃)-CH(OH)-CH₂-O-PO(OH)₂
CH (OH)=C (CH₃) -CH (OH) -CH₂-OH,
(CH₃) ₂ CH-CO-CH₂-O-PO (OH)₂,
(CH₃) ₂ CH-CO-CH₂-OH,
(CH₃) ₂CH-CH(OH)-CH₂-O-PO(OH)₂,
(CH₃) ₂ CH-CH (OH) -CH₂-OH.
